(19) Europäisches Patentamt
European Patent Office
Office européen des brevets

(11) **EP 2 116 223 B1**

(12) **EUROPEAN PATENT SPECIFICATION**

(45) Date of publication and mention of the grant of the patent:
**09.12.2015 Bulletin 2015/50**

(21) Application number: **07860431.1**

(22) Date of filing: **27.12.2007**

(51) Int Cl.:
*A61K 8/67* (2006.01)     *A61K 8/06* (2006.01)
*A61K 8/55* (2006.01)     *A61K 8/97* (2006.01)
*A61Q 19/00* (2006.01)     *B01J 13/00* (2006.01)

(86) International application number:
**PCT/JP2007/075210**

(87) International publication number:
**WO 2008/081905 (10.07.2008 Gazette 2008/28)**

(54) **DISPERSION COMPOSITION, COSMETIC PREPARATION FOR SKIN CARE, AND METHOD FOR PRODUCING DISPERSION COMPOSITION**

DISPERSIONSZUSAMMENSETZUNG, KOSMETISCHE ZUBEREITUNG ZUR HAUTPFLEGE UND VERFAHREN ZUR HERSTELLUNG DER DISPERSIONSZUSAMMENSETZUNG

COMPOSITION DE DISPERSION, PRÉPARATION COSMÉTIQUE POUR UN SOIN DE LA PEAU, ET PROCÉDÉ DE FABRICATION D'UNE COMPOSITION DE DISPERSION

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **05.01.2007 JP 2007000679**
            **27.06.2007 JP 2007169635**

(43) Date of publication of application:
**11.11.2009 Bulletin 2009/46**

(73) Proprietor: **FUJIFILM Corporation**
**Minato-ku**
**Tokyo 106-8620 (JP)**

(72) Inventors:
• **KAWABUCHI, Tatsuo**
**Tokyo 1068620 (JP)**
• **KUBO, Toshiaki**
**Ashigarakami-gun, Kanagawa 258-8577 (JP)**
• **OGAWA, Manabu**
**Ashigarakami-gun, Kanagawa 258-8577 (JP)**
• **SUZUKI, Keiichi**
**Ashigarakami-gun, Kanagawa 258-8577 (JP)**
• **TASHIRO, Tomoko**
**Ashigarakami-gun, Kanagawa 258-8577 (JP)**

• **SUDO, Yukio**
**Ashigarakami-gun, Kanagawa 258-8577 (JP)**

(74) Representative: **Morpeth, Fraser Forrest**
**Fujifilm Imaging Colorants Limited**
**Intellectual Property Group**
**P.O. Box 42**
**Hexagon Tower**
**Blackley, Manchester M9 8ZS (GB)**

(56) References cited:
EP-A1- 1 864 578      WO-A1-2005/110370
WO-A2-2004/112689    JP-A- 2000 106 844
JP-A- 2000 106 844     JP-A- 2001 524 087
JP-A- 2004 518 645     JP-A- 2005 506 841
JP-A- 2007 197 328     JP-A- 2007 269 749
JP-A- 2007 326 829     JP-A- 2008 013 751
US-A1- 2003 129 290

Remarks:
The file contains technical information submitted after the application was filed and not included in this specification

**Description**

TECHNICAL FIELD

The present invention relates to a dispersion composition, a cosmetic preparation for skin care, and a method for producing the dispersion composition, in particular, to a dispersion composition in which a carotenoid-containing oily component is dispersed in an aqueous composition, a cosmetic preparation for skin care therewith, and a method for producing the dispersion composition.

BACKGROUND ART

Carotenoids are naturally-found yellow to red-colored terpenoid colorants and may be found in plants, algae, and bacteria. Astaxanthins (containing astaxanthin and esters thereof as well), which are one of the carotenoids found widely distributed throughout the animal and plant kingdoms in the natural world, are used mainly as revivers for farmed fish and commercial poultry. Furthermore, it is well known that astaxanthin has functions including an antioxidant effect, an anti-inflammatory effect (see, for example, Japanese Patent Application Laid-Open (JP-A) Nos. 2-49091 and 9-143063), a skin aging inhibiting effect (see, for example, JP-A No. 5-155736), a preventive effect against spots or lines (see, for example, JP-A No. 2005-47860) and so on. Accordingly, it is under study and in practice to add astaxanthin to raw materials of foodstuffs, cosmetics and drugs and processed goods thereof.

When carotenoids are added to foodstuffs, cosmetics, drugs and other processed goods, in many cases, the carotenoids are added as an emulsion composition with high dispersibility. However, a naturally-derived carotenoid is structurally unstable and, when the particle diameters of emulsion particles are within a satisfactory range, is difficult to maintain high dispersion stability over a relatively long period.

In order to overcome this problem, a technique that investigated the dispersion stability of carotenoid colorants is described in, for example, JP-A No. 9-328419 and Japanese National Phase Publication No. 2005-506841.

However, even with this technique, in an aqueous dispersion containing a carotenoid, dispersibility, color, and properties deteriorate with time. That is, the stability of a dispersion composition containing a carotenoid has been difficult to maintain over a desired period of time.

DISCLOSURE OF THE INVENTION

Means for Solving the Problems

The present invention was achieved in view of the above situations and provides a dispersion composition and a method for producing the dispersion composition.

The invention provides a dispersion composition containing a carotenoid-containing oily component, wherein the dispersion composition is obtained by mixing an aqueous dispersion containing emulsion particles containing the carotenoid-containing oily component and containing a phospholipid or a derivative thereof; and an aqueous composition containing at least one specific ascorbic acid derivative and an oily component that is 20% by mass or less relative to the mass of the whole dispersion composition, and wherein the dispersion composition contains emulsion particles having an average particle diameter of 200 nm or less.

A second aspect of the invention is a cosmetic preparation for skin care, which contains the dispersion composition.

A third aspect of the invention provides a method for producing a dispersion composition containing a carotenoid-containing oily component, the producing method comprising; mixing a carotenoid-containing oily component and a phospholipid or a derivative thereof with an aqueous phase to obtain an aqueous dispersion containing emulsion particles; and mixing the aqueous dispersion and an aqueous composition containing at least one specific ascorbic acid derivative and an oily component that is 20% by mass or less relative to the mass of the whole dispersion composition to obtain a dispersion composition comprising emulsion particles having an average particle diameter of 200 nm or less. composition to the range of 5 to 7.5.

BEST MODE FOR CARRYING OUT THE INVENTION

A dispersion composition of the invention is a dispersion composition containing astaxanthin, wherein the dispersion composition is obtained by mixing an aqueous dispersion containing emulsion particles containing the carotenoid-containing oily component and a phospholipid or a derivative thereof and an aqueous composition containing at least one specific ascorbic acid derivative and an oily component that is 20% by mass or less relative to the mass of the whole dispersion composition, and wherein the dispersion composition has emulsion particles having an average particle diameter of 200 nm or less.

A cosmetic preparation for skin care of the invention contains the dispersion composition.

The carotenoid is astaxanthin, and is preferably an extract of hematococcus alga.

The ascorbic acid derivative is at least one selected from magnesium ascorbyl phosphate, sodium ascorbyl phosphate.

The phospholipid or a derivative thereof is preferably in the range of 0.001 to 20% by mass related to the mass of the whole aqueous dispersion.

In the dispersion composition, the aqueous dispersion composition may further contain tocopherol.

In what follows, the invention will be detailed. In the specification, "to (~)" shows a range that includes numerical values described before and after the "to (~)", respectively, as the minimum and maximum values.

In the beginning, an aqueous dispersion involving the invention will be described.

The aqueous dispersion involving the invention contains emulsion particles containing astaxanthin and a phospholipid or a derivative thereof.

A content of the carotenoid in the aqueous dispersion is preferably in the range of 0.1 to 10% by mass, more preferably in the range of 0.2 to 5% by mass, and still more preferably in the range of 0.5 to 2% by mass relative to the mass of the aqueous dispersion, from the viewpoint of more excellently exerting a functional effect of containing carotenoid when an aqueous dispersion is formed.

The carotenoid used in the invention is preferably oily one at normal temperature from the viewpoint of making an organic medium used at the time of dispersion as small as possible. Astaxanthin has an antioxidant effect, an antiinflammatory effect, a skin aging inhibiting effect and a whitening effect and is known as a colorant in the range from yellow to red.

Astaxanthin is a red colorant having absorption maximum at 476 nm (ethanol) and 468 nm (hexane), and belongs to exanthophylls as one kind of carotenoid (Davies, B. H.: In "Chemistry and Biochemistry of Plant Pigments", T. W. Goodwin ed., 2nd ed., 38-165, Academic Press, NY, 1976). The chemical structure of astaxanthin is 3,3'-dihydroxy-$\beta,\beta$-carotene-4,4'-dione ($C_{40}H_{52}O_4$, molecular weight 596.82).

In the astaxanthin, three isomers of 3S,3S'-form, 3S,3R'-form (meso form) and 3R,3R'-form are present depending on a steric configurations of hydroxyl groups at 3 (3')-position of a ring structure present at both ends of the molecule. Additionally, cis- and trans-isomers of a conjugated double bond at the molecular center are present. For example, there are all cis-, 9-cis and 13-cis isomers.

The hydroxyl group at a 3(3')-position can form an ester with fatty acid. Astaxanthin obtained from Euphausiacea are 3S,3S'-form when obtained from a diester having two fatty acids bonded thereto (Yamaguchi, K., Miki, W., Toriu, N., Kondo, Y., Murakami, M., Konosu, S., Satake, M., and Fujita, T.: The composition of carotenoid colorants in the antrarctic krill Euphausia superba, Bull. Jap. Sos. Sci. Fish., 1983, 49, p.1411-1415), and Astaxanthin obtained from H. pluvialis has a 3S,3S'-form and contains a large amount of a monoester having one fatty acid bonded thereto (Renstrom, B., Liaaen-Jensen, S.: Fatty acids of some estrified carotenols, Comp. Biochem. Physiol. B, Comp. Biochem., 1981, 69, p.625-627).

Furthermore, astaxanthin obtained from Rhaffia Rhodozyma is 3R,3R'-form (Andrewes, A.G., Starr, M. P.: (3R,3'R)-Astraxanthin from the yeast Phaffarhodozyma, Phytochem., 1976, 15, p.1009-1011), and has a structure opposite 3S,3S'-form generally found naturally. This is present in a free form which does not form an ester with fatty acid (Andrewes, A.G., Phaffia, H. J., Starr, M. P.: Carotenids of Phaffia rhodozyma, a red pigmented fermenting yeast, Phytochem., 1976, 15, p.1003-1007).

Astaxanthin and its ester form were first separated from a lobster (Astacus gammarus L.) by Kuhn et al., and its estimated structure was disclosed (Kuhn, R., Soerensen, N. A.: The coloring matters of the lobster (Astracus gammarus L.), Z. Angew. Chem., 1938, 51, p.465-466). Since then, it has been clarified that astaxanthin is widely distributed in nature, is generally present as in the astaxanthin fatty acid ester form, and is also present as astaxanthin protein (ovorubin and crustacyanin) bonded to protein in crustacean (Cheesman, D. F.: Ovorubin, a chromoprotein from the eggs of the gastropod mollusc Pomacea canaliculata, Proc. Roy. Soc. B, 1958, 149, p.571-587).

The astaxanthin may be contained in the emulsion composition of the invention as an astaxanthin-containing oil separated and extracted from natural products containing astaxanthin. Examples of such an astaxanthin-containing oil include extracts obtained by culturing red yeast Phaffia, green alga Haematococcus, marine bacteria or the like and extracting from its culture, and extracts from antactic Euphausiacea and the like.

Astaxanthin has two hydroxyl groups in a molecule. It is known different in that a hematococcus alga extract (colorant derived from hematococcus alga) is mainly made of monoesters and a colorant derived from krill is mainly made of diesters.

Astaxanthins that can be used in the invention may be the above-described extracts, products by appropriate purification of those extracts according to need, and synthetic products. Astaxanthins that are particularly preferably, from the point of view of quality and productivity, are products extracted from Haematococcus alga (called Haematococcus alga extracts).

Specific examples that may be given of products derived from Haematococcus alga extract that can be used in the invention include Haematococcus pluvialis, Haematococcus lacustris, Haematococcus capensis, Haematococcus droebakensis and Haematococcus zimbabwiensis.

Various methods may be used as the method for culturing of Haematococcus alga that can be used in the invention, such as the methods disclosed in JP-A-8-103288. The methods are not particularly limited, as long as the cells have undergone morphological change from the vegetative cells to cyst cells, which are as dormant cells.

Haematococcus alga extracts that can be used in the invention are obtained by crushing, as required, cell walls of the above raw materials by a method described in, for example, JP-A No. 5-68585 and adding an extracting solvent such as an organic solvent such as acetone, ether, chloroform or alcohol (such as ethanol, methanol), or carbon dioxide in a supercritical state, followed by extraction.

The Haematococcus alga extracts may contain astaxanthin as a colorant pure component similar to the colorant described in JP-A-2-49091, and may contain the ester in an amount of generally 50% by mol or more, preferably 75% by mol or more, and more preferably 90% by mol or more.

In the invention, commercially available Haematococcus alga extracts may be used, and examples thereof include ASTOTS-S, ASTOTS-2.5 O, ASTOTS-5 O and ASTOTS-10 O, manufactured by Takedashiki Co., Ltd.; AstaREAL oil 50F and AstaREAL oil 5F, manufactured by Fuji Chemical Industry Co., Ltd.; and BioAstin SCE7, a product of Toyo Koso Kagaku Co., Ltd.

In the invention, the content of the astaxanthins as a colorant pure component in Haematococcus alga extracts may preferably from 0.001 % to 50% by mass, and more preferably from 0.01 % to 25% by mass.

Furthermore, a blending amount of the astaxanthin-containing oil in the aqueous dispersion is preferably in the range of 0.001 to 20% by mass and more preferably in the range of 0.1 to 10% by mass relative to the mass of the aqueous dispersion, from the viewpoint of stability of the dispersion.

Phospholipid as used in the invention means one group of conjugated lipids, which group includes a phosphoric acid esters and fatty acid esters comprising a fatty acid, an alcohol, phosphorus and a nitrogen compound, and includes glycerophospholipids other than glycerin, and sphingophospholipids comprising sphingosin.

Examples of glycerophospholipids used in the invention include phosphatidic acid, bisphosphatidic acid, lecithin (phosphatidylcholine), phosphatidylethanolamine, phosphatidylmethylethanolamine, phosphatidylserin, phosphatidylinositol, phosphatidylglycerol, diphosphatidylglycerol, sphingomyelin and the like, and additionally include various lecithins including lecitines derived from plants such as soybeans, corn, peanuts, rapeseeds and wheat; lecithins derived from animals such as egg yolk and cows; and lecithins derived from microorganisms such as Escherichia coli, all of which contain a phospholipid component such as those described above. Lecithin that is a mixture thereof or hydrogenated lecithin may be used as well. The origin of those phospholipids is not particularly limited and plant oil such as soybean oil or the like and products derived from animals such as egg yolk or the like, may be used, and purified ones are particularly preferably.

Furthermore, in the invention, phospholipid may include lysolecithin glycerophospholipid. Lysolethicine is a compound having, as the result of an enzymatic decomposition, one fatty acid residue in one molecule, that is, lysolecithin as well is contained.

Lysolecithin is obtained by hydrolysis of lecithin in the presence of an acid or alkali catalyst, and may also be obtained by hydrolysis of lecithin using phospholipase $A_1$ or $A_2$.

Examples of lysolecithin include lisophosphatidic acid, lysophosphatidylglycerin, lysophosphatidylinositol, lys-ophosphatidylethanolamine, lysophosphatidylmethylethanolamine, lysophosphatidylcholine (lysolecithin), lysophos-phatidylserine and the like.

Furthermore, the glycerophospholipid represented by the lecithin in the invention may use hydrogenated or hydroxylated products such as hydrogenated lecithins, enzymatically decomposed lecithins, enzymatically decomposed and hydrogenated lecithins and hydroxylecithins.

The hydrogenation may be carried out by, for example, reacting lecithin with hydrogen in the presence of a catalyst, thereby the unsaturated bond in the fatty acid moiety is hydrogenated. The hydrogenation improves oxidation stability of lecithin.

Hydroxylation involves hydroxylation of an unsaturated bond(s) in the fatty acid moiety by heating lecithin together with high concentration of hydrogen peroxide and an organic acid such as acetic acid, tartaric acid or butyric acid. Hydroxylation improves the hydrophilicity of lecithin.

In the invention, lecithin is particularly preferred from the viewpoint of emulsification stability.

Examples of commercially available lecithin products include LECION SERIES and LECIMAL EL (trade name, manufactured by Riken Vitamin Co., Ltd.) and so on.

Lecithin having a purity of 60% by mass or more is industrially used as lecithin. In the invention, lecithin having a purity of 80% by mass or more generally called "high purity lecithin" is preferred, and lecithin having a purity of 90% by mass or more is more preferred.

The lecithin purity (% by mass) is obtained by subtracting the weights of toluene-insoluble substances and acetone-soluble substances, utilizing the property that lecithin tends to dissolve in toluene and does not dissolve in acetone. High purity lecithin has high lipophilicity as compared with lysolecithin, and it is therefore because compatibility between lecithin and an oily component is increased, thereby improving emulsion stability.

The phospholipid used in the invention may be used alone or as mixtures of two or more thereof.

A content of the phospholipid in the aqueous dispersion in the invention is preferably in the range of 0.001 to 20% by mass and more preferably in the range of 0.001 to 10% by mass relative to the aqueous dispersion from the viewpoint of the emulsification stability.

An aqueous emulsifier that may be used in the aqueous dispersion involving the invention is not particularly limited as long as the emulsifier is dissolved in an aqueous medium. For example, a nonionic surfactant having the HLB of 10 or more, preferably of 12 or more is preferred. When the HLB is less than 10, emulsifying power may be insufficient in some cases. The HLB is preferred to be 16 or less from the viewpoint of the emulsification stability.

The HLB used herein is the balance of hydrophilicity-hydrophobicity generally used in the field of surfactants, and a generally used calculation formula g, such as Kawakami's equation formula, can be used. The following Kawakami's equation is adopted in the invention.

$$HLB = 7 + 11.7 \log (M_w + M_o)$$

wherein $M_w$ is the molecular weight of the hydrophilic group, and $M_o$ is the molecular weight of the hydrophobic group.

HLB values described in brochures and the like may be used.

As is apparent from the above formula, a surfactant having a desired HLB value can be obtained by utilizing additive properties of HLB.

Emulsifiers that can be used in the invention is not particularly limited and, nonionic surfactants are preferred. Examples of nonionic surfactants include glycerin fatty acid ester, organic acid monoglyceride, polygrycerin fatty acid ester, propylene glycol fatty acid ester, polyglycerin condensed licinoleic acid ester, sorbitan fatty acid ester, sucrose fatty acid ester and the like. Polyglycerin fatty acid ester, sorbitan fatty acid ester and sucrose fatty acid ester are preferred. The emulsifiers are not always required to be highly purified, by distillation or the like, and may be a reaction mixture.

The polyglycerin fatty acid ester used in the invention is an ester of a polyglycerin having an average degree of polymerization of 2 or more, preferably from 6 to 15, and more preferably from 8 to 10, and a fatty acid having from 8 to 18 carbon atoms such as caprylic acid, capric acid, lauric acid, myristic acid, palmitic acid, stearic acid, oleic acid and linoleic acid. Preferred examples of the polyglycerin fatty acid ester include hexaglycerin monooleic acid ester, hexaglycerin monostearic acid ester, hexaglycerin monopalmitic acid ester, hexaglycerin monomyristic acid ester, hexaglycerin monolauric acid ester, decaglycerin monooleic acid ester, decaglycerin monostearic acid ester, decaglycerin monopalmitic acid ester, decaglycerin monomyristic acid ester, decaglycerin monolauric acid ester and the like. Those polyglycerin fatty acid esters may be used alone or as mixtures thereof. Examples that may be given of suitable commercially available products include NIKKOL DGMS, NIKKOL DGMO-CV, NIKKOL DGMO-90V, NIKKOL DGDO, NIKKOL DGMIS, NIKKOL DGTIS, NIKKOL Tetraglyn 1-SV, NIKKOL Tetraglyn 1-O, NIKKOL Tetraglyn 3-S, NIKKOL Tetraglyn 5-S, NIKKOL Tetraglyn 5-O, NIKKOL Hexaglyn 1-L, NIKKOL Hexaglyn 1-M, NIKKOL Hexaglyn 1-SV, NIKKOL Hexaglyn 1-O, NIKKOL Hexaglyn 3-S, NIKKOL Hexaglyn 4-B, NIKKOL Hexaglyn 5-S, NIKKOL Hexaglyn 5-O, NIKKOL Hexaglyn PR-15, NIKKOL Decaglyn 1-L, NIKKOL Decaglyn 1-M, NIKKOL Decaglyn 1-SV, NIKKOL Decaglyn 1-50SV, NIKKOL Decaglyn 1-ISV, NIKKOL Decaglyn 1-O, NIKKOL Decaglyn 1-OV, NIKKOL Decaglyn 1-LN, NIKKOL Decaglyn 2-SV, NIKKOL Decaglyn 2-ISV, NIKKOL Decaglyn 3-SV, NIKKOL Decaglyn 3-OV, NIKKOL Decaglyn 5-SV, NIKKOL Decaglyn 5-HS, NIKKOL Decaglyn 5-IS, NIKKOL Decaglyn 5-OV, NIKKOL Decaglyn 5-O-R, NIKKOL Decaglyn 7-S, NIKKOL Decaglyn 7-O, NIKKOL Decaglyn 10-SV, NIKKOL Decaglyn 10-IS, NIKKOL Decaglyn 10-OV, NIKKOL Decaglyn 10-MAC and NIKKOL Decaglyn PR-20, manufactured by Nikko Chemicals Co., Ltd.; RYOTO-polyglyester L-7D, L-10D, M-10D, M-7D, P-8D, SWA-10D, SWA-15D, SWA-20D, S-24D, S-28D, O-15D, O-50D, B-70D, B-100D, ER-60D, manufactured by Mitsubishi-Kagaku Foods Corporation; SUNSOFT Q-17UL, SUNSOFT Q-14S and SUNSOFT A-141C, manufactured by Taiyo Kagaku Co., Ltd.; and POEM DO-100 and POEM J-0021, manufactured by Riken Vitamin Co., Ltd.

The sorbitan fatty acid ester used in the invention has preferably 8 or more carbon atoms, and more preferably 12 or more carbon atoms, in the fatty acid moiety. Preferred examples of the sorbitan fatty acid ester include sorbitan monocaprylate, sorbitan monolaurate, sorbitan monostearate, sorbitan sesquistearate, sorbitan tristearate, sorbitan isostearate, sorbitan sesquiisostearate, sorbitan oleate, sorbitan sesquioleate and sorbitan trioaleate, and the like. Those sorbitan fatty acid esters can be used alone or as mixtures thereof. Examples that may be given of suitable commercially available manufactured by the sorbitan fatty acid ester include NIKKOL SL-10, SP-10V, SS-10V, SS-10MV, SS-15V, SS-30V, SI-10RV, SI-15RV, SO-10V, SO-15MV, SO-15V, SO-30V, SO-10R, SO-15R, SO-30R and SO-15EX, manufactured by Nikko Chemicals Co., Ltd.; SORGEN 30V, 40V, 50V, 90 and 110, manufactured by Daiichi-Kogyo Seiyaku Co., Ltd.; and the like.

The sucrose fatty acid ester used in the invention has preferably 12 or more, and more preferably from 12 to 20, carbon atoms in the fatty acid moiety. Preferred examples of the sucrose fatty acid ester include sucrose dioleate, sucrose distearate, sucrose dipalmitate, sucrose dimyristate, sucrose dilaurate, sucrose monooleate, sucrose monostearate, sucrose monopalmitate, sucrose monomyristate and sucrose laurate, and the like. In the invention, those sucrose fatty acid esters may be used alone or as mixtures thereof. Examples that may be given of suitable commercially available product of the sucrose fatty acid ester include RYOTO-sugar ester S-070, S-170, S-270, S-370, S-370F, S-570, S-770, S-970, S-1170, S-1170F, S-1570, S-1670, P-070, P-170, P-1570, P-1670, M-1695, O-170, O-1570, OWA-1570, L-195, L-595, L-1695, LWA-1570, B-370, B-370F, ER-190, ER-290 and POS-135, manufactured by Mitsubishi-Kagaku Foods Corporation; DK ester SS, F160, F140, F110, F90, F70, F50, F-A50, F-20W, F-10 and F-A10E, and COSMELIKE B-30, S-10, S-50, S-70, S-110, S-160, S-190, SA-10, SA-50, P-10, P-160, M-160, L-10, L-50, L-160, L-150A, L-160A, R-10, R-20, O-10 and O-150, manufactured by Daiichi-Kogyo Seiyaku Co., Ltd, and the like.

An addition amount of the emulsifier is preferably in the range of 0.1 to 50% by mass, more preferably in the range of 0.5 to 20% by mass, and further more preferably in the range of 1 to 15% by mass relative to the aqueous dispersion. When the emulsifier is added 0.1% by mass or more, an emulsion having finer particle diameters is obtained and the stability of the emulsion is sufficiently secured. When the emulsifier is added in an amount of 50% by mass or less, forming of the emulsifier may be controlled to within a suitable range.

The aqueous dispersion preferably further contains tocopherol as an oily component from the viewpoint of inhibiting the carotenoids from oxidizing.

Usable tocopherol is not particularly limited and is selected from a group of compounds consisting of tocopherol and derivatives thereof.

Examples of the group of compounds selected from tocopherol and its derivatives include tocopherol and its derivatives such as dl-α-tocopherol, dl-β-tocopherol, dl-γ-tocopherol, dl-δ-tocopherol, dl-α-tocopherol acetate, dl-α-tocopherol nicotinate, dl-α-tocopherol linoleate, dl-α-tocopherol succinate; and α-tocotrienol, β-tocotrienol, γ-tocotrienol and δ-tocotrienol; and the like. These compounds are frequently used in the state of a mixture, and can be used in the state called "extracted tocopherol", "mixed tocopherol" and the like.

A content of tocopherol in the aqueous dispersion is not particularly limited. From the viewpoint of inhibiting carotenoids from oxidizing, the content of tocopherol may be at a ratio preferably in the range of 0.1 to 5, more preferably in the range of 0.2 to 3, and still more preferably in the range of 0.5 to 2 relative to a mass of carotenoid.

In the aqueous dispersion involving the invention, glycerin may be preferably contained from the viewpoint of making an average particle diameter of emulsion particles in the aqueous dispersion smaller and of enabling to stably maintain the particle diameter as small over a long term.

In this case, a content of glycerin is preferably in the range of 10 to 60% by mass, more preferably in the range of 20 to 55% by mass and still more preferably in the range of 30 to 50% by mass relative to a mass of the whole aqueous dispersion, from the viewpoint of dispersion stability and antiseptic properties.

Furthermore, the aqueous dispersion may preferably contain an antioxidant.

Examples of an usable antioxidant include, but are not particularly limited to, a group of compounds consisting of polyphenols, radical scavengers, the tocopherols aforementioned and the like.

As the antioxidant usable in the invention, a hydrophilic antioxidant and/or a lipophilic antioxidant may be used singularly or in a combination thereof.

A content of the antioxidant in the aqueous dispersion in the invention is generally in the range of 0.1 to 10% by mass, preferably in the range of 0.2 to 5% by mass and more preferably in the range of 0.5 to 2% by mass.

Ascorbic acid or an ascorbic acid derivative generally usable as the antioxidant is not contained in the aqueous dispersion. These compounds are, as will be described below, contained in an aqueous composition for mixing with the aqueous dispersion.

Examples of the group of compounds consisting of polyphenols as the antioxidant usable in the invention include flavonoids (catechin, anthocyanin, flavone, isoflavone, flavane, flavanone and rutin), phenolic acids (chlorogenic acid, ellagic acid, gallic acid and propyl gallate), lignans, curcumins and coumarins. Those compounds are contained, in a significant amount, in the following naturally-derived extracts, and therefore can be used in the state of an extract.

Examples of naturally-derived extracts include a glycyrrhiza extract, a cucumber extract, a Mucuna birdwoodiana stem extract, a gentian root extract, a Geranium thunbergii extract, a cholesterol and its derivatives, a Crataegus cuneata fruit extract, a peony root extract, a gingko extract, a Phellodendron bark extract, a carrot extract, a rugosa rose (Maikai) extract, a Cassia mimosoides extract, a Tormentilla root extract, a parsley extract, a Paeonia suffruticosa root extract, a Japanese quince extract, a Melissa officinalis leaf extract, an alnus firma fruit extract, a Saxifraga sarmentosa extract, a rosemary leaf extract, a lettuce leaf extract, a tea extract (oolong tea, black tea, green tea or the like), a microbial fermentation metabolic product and a Rakanka (Momordica grosvenori Swingle) extract, and the like. Of those polyphenols, particularly preferred are catechin, a rosemary extract, glucosyl rutin, ellagic acid and gallic acid.

Antioxidants usable in the invention may be appropriately selected from commercially available products. Examples thereof include ellagic acid (Wako Pure Chemical Industries, Ltd., and the like), rosemary extracts (trade name: RM-21A, RM-21E, Mitsubishi-Kagaku Foods Corporation, and the like), catechin (trade name: SANKATOL W-5, No. 1, Taiyo Kagaku Co., Ltd., and the like), sodium gallate (trade name: SANKATOL, Taiyo Kagaku Co., Ltd., and the like), and rutins, glurcosylrutins and enzymatically decomposed rutins (trade name: Rutin K-2, P-10, Kiriya Chemical Co., Ltd.; trade name: αG Rutin, Hayashibara Biochemical Labs., Inc.; and the like).

A group of the radical trapping agent usable in the invention is an additive that suppresses generation of radicals, and further has a role of rapidly trapping radicals, thereby stopping a chain reaction from taking place (Source: Oil Chemistry Handbook, 4th edition, Japan Oil Chemists' Association, 2001). Known methods of directly confirming the function as a radical trapping agent include a method in which the radical trapping agent is mixed with a reagent, and the state of trapping the radial is measured with a spectrophotomer or by ERS

(electron spin resonance). Those methods use DPPH (1,1-diphenyl-2-picrylhydrazyl) or a garbinoxyl radial as the reagent.

In the invention, a compound that gives a time required to increase the peroxide value (POV) of an oil or fat to 60 meq/kg, utilizing an auto-oxidation reaction of oil or fat under the following experimental conditions, that is 2 times or more, preferably 5 times o more, that without the agent is defined as a "radial trapping agent".

Oil or fat: Olive oil
Amount of specimen added: 0.1% by mass based on mass of oil or fat
Test method: Sample was heated to 190°C, POV was measured with the passage of time by a conventional method, and time required to reach a POV of 60 meq/kg was calculated.

Compounds that may be used as the radical trapping agent of the invention may be any compound as long as it functions as a radical trapping agent in various antioxidants described in "Theory and Fact of Antioxidant" (Kajimoto, San Shobo, 1984) and "Antioxidant Handbook" (Saruwatari, Nishino and Tabata, Taiseisha, 1976), and specific examples thereof include compounds having phenolic OH, amine compounds such as phenylenediamine, and oil-solubilized derivatives of ascorbic acid and erythorbic acid.

In what follows, examples of preferable compound are cited without restricting the invention thereto.

Examples of the compound having a phenolic OH include a guaiac, nordihydroguaiaretic acid (NDGA), gallic acid esters, BHT (butylhydroxytoluene), BHA (butylhydroxyanisole), tocopherols, bisphenols and so on. Examples of gallic acid esters include propyl gallate, butyl gallate and octyl gallate.

As an amine compound, phenylene diamine is cited, and diphenyl-p-phenylenediamine or 4-amino-p-diphenylamine is more preferred.

As other oily component in the aqueous dispersion, other components that are usually used as a UV-absorbent, an antioxidant, an antiinflammatory agent, a moisturizing agent, a hair-protecting agent, a dispersant, a solvent, a whitening agent, an anti-spot agent, a cell activator, an emollient agent, a kelatolytic drug, an antistatic agent, vitamins, a metabolic syndrome improver, an antihypertensive drug, a tranquilizer or the like may be used. Examples thereof include, for example, oil or fats such as olive oil, camellia oil, macadamia nut oil, castor seed oil or the like, hydrocarbons such as fluid paraffin, paraffin, vaseline, ceresin, microcrystalline wax, squalane or the like, waxes such as carnauba wax, candelilla wax, jojoba wax, bee wax, lanolin or the like, esters such as isopropyl myristate, 2-octyldodecyl myristate, cetyl 2-ethylhexanoate, diisostearyl mallate or the like, fatty acids such as palmitic acid, stearic acid, isostearic acid or the like, higher alcohols such as cetyl alcohol, stearyl alcohol, isostearyl alcohol, 2-octyldodecanol or the like, silicone oil such as methylpolysiloxane, methylphenylpolysiloxane or the like, fatty acid esters of glycerin, and, others including polymers, oil-soluble colorants, oil-soluble proteins and so on. Furthermore, various kinds of plant-derived oils or animal-derived oils that are mixtures thereof may be included as well. Examples of other oily component that is preferably used in the invention include vitamin Es (other than the tocopherol mentioned above, tocotrienol and so on), coenzyme Qs, $\overline{\omega}$-3 oil or fats (oil or fat containing EPA, DHA and linolenic acid).

A content of astaxanthin in the invention is preferably in the range of 0.1 to 30% by mass, more preferably in the range of 1 to 20% by mass and still more preferably in the range of 5 to 15% by mass relative to a mass of the aqueous dispersion, from the viewpoint of making diameters of emulsified particles finer and emulsification stability.

An organic solvent is preferably added to the aqueous dispersion in the invention in order to dissolve foregoing various oily components and other components and to make an average particle diameter of emulsion in the aqueous dispersionparticles finer.

Such the organic solvent is preferably water-soluble, and examples thereof include, for example, methanol, ethanol, isopropanol, acetone, tetrahydrofuran, acetonitrile and the like, and mixtures thereof. Among these, ethanol is preferred from the viewpoint of applying to foodstuffs, cosmetic preparations or the like.

A content of the organic solvent relative to the aqueous dispersion may be, for example, in the range of 0.1 to 20% by mass and preferably in the range of 0.5 to 10% by mass relative to a mass of the aqueous dispersion.

Emulsion particles in the aqueous dispersion have a volume average particle diameter (median diameter) of 200 nm or less, preferably of 150 nm or less and more preferably of 100 nm or less, from the viewpoint of the transparency. Particles having such an average particle diameter may be readily obtained by mixing the components of the aqueous dispersion at an amount ratio mentioned above.

The particle diameter varies depending on various factors such as kinds and usage amounts of added components, an emulsification condition in a producing method (shearing force, temperature, or pressure), usage amounts of additives, an oil phase to water phase ratio, a usage amount of a surfactant or the like. However, when a particle diameter satisfies the invention, there is no practical problem.

The particle size of the emulsion composition of the invention can be measured with a commercially available particle size distribution measuring device. Optical microscopy, confocal laser microscopy, electron microscopy, atomic force microscopy, static light scattering method, laser diffraction method, dynamic light scattering method, centrifugal precipitation method, electric pulse measurement method, chromatography method, ultrasonic damping method and the like are known as particle size distribution measurement methods of an emulsion, and devices corresponding to the respective principle are commercially available.

From particle size range in the invention and ease of measurement, a dynamic light scattering method is preferred in the emulsion particle size measurement in the invention. Commercially available measurement devices using dynamic light scattering include Nanotrac UPA (Nikkiso Co., Ltd.), dynamic light scattering particle size distribution measuring device LB-550 (Horiba, Ltd.) and fiber-optics particle size analyzer FPAR-1000 (Otsuka Electronics Co., Ltd.). A measurement temperature may be a temperature that is usually used to measure a particle diameter and is preferably 20°C.

A particle diameter in the invention is a value that is measured at 20°C with a dynamic light scattering particle size distribution analyzer.

As an aqueous medium that constitutes the aqueous dispersion involving the invention, water is mainly cited. Any one of distilled water, ion-exchanged water, pure water and so on may be used.

An aqueous dispersion containing a carotenoid-containing oily component in the invention may be obtained, but is not limited to by, for example, a) dissolving an aqueous emulsifier in an aqueous medium such as water to obtain an aqueous phase, b) mixing and dissolving the carotenoid, tocopherol, phospholipid, and, as required, other oil or fat to obtain an oil phase, and c) mixing an aqueous phase and an oil phase under stirring to emulsify and disperse.

When the aqueous dispersion is emulsified and dispersed, at least two kinds of emulsifying devices are particularly preferably used together in such a manner that an ordinary emulsifying device such as a stirrer, an impeller stirrer, a homomixer, a continuous path type shearing device or the like, which makes use of a shearing action, is used to emulsify, followed by passing a high-pressure homogenizer. When the high-pressure homogenizer is used, an emulsion may be rendered liquid drops of more uniform fine particles. Furthermore, the emulsification and dispersion may be applied a plurality of times to obtain liquid drops of more uniform particle diameters.

Examples of high-pressure homogenizer include a chamber type high-pressure homogenizer that has a chamber where a flow path of a processing liquid is fixed, and a homogeneous valve high-pressure homogenizer having a homogeneous valve. Among these, the homogeneous valve high-pressure homogenizer may readily control a width of a flow path of the processing liquid, thereby pressure and a flow rate at the time of operation may be optionally set, that is, an operational range thereof is wide; accordingly, the homogeneous valve high-pressure homogenizer is preferably used in the invention. The chamber type high-pressure homogenizer that is low in the degree of freedom of operation but is easy to form a structure that heightens pressure is preferably used in applications that necessitate ultrahigh pressure.

Examples of the chamber type high-pressure homogenizer include MICROFLUIDIZER (manufactured by Microfluidics Inc.,), NANOMIZER (manufactured by Yoshida Kikai Kogyo Co., Ltd.), ULTIMIZER (manufactured by Sugino Machine Ltd.,) and so on.

Examples of the homogeneous high-pressure homogenizer include GOLIN HOMOGENIZER (manufactured by APV Inc.,), LANIER HOMOGENIZER (manufactured by Lanier Inc.,), HIGH-PRESSURE HOMOGENIZER (manufactured by Niro Soavi), HOMOGENIZER (manufactured by SANWA MECHINERY TRADING CO., LTD.), HIGH-PRESSURE HOMOGENIZER (manufactured by Izumi Food Machinery Co., Ltd.), ULTRAHIGH-PRESSURE HOMOGENIZER (manufactured by IKE Co., Ltd.) and so on.

In the invention, pressure of the high-pressure homogenizer is set preferably at 50 MPa or more, more preferably at 50 to 250 MPa, and still more preferably at 100 to 250 MPa to treat. An emulsion liquid that is an emulsified and dispersed composition is cooled through any cooler within 30 sec immediately after pass of the chamber, and preferably within 3 sec, preferably from the viewpoint of maintaining particle diameters of dispersed particles.

In the following, the aqueous composition of the invention will be described. The aqueous composition of the invention contains at least one ascorbic acid derivative selected from magnesium or sodium ascorbyl phosphate.

In the invention, it is contained in the aqueous composition; accordingly, when the aqueous composition and an aqueous dispersion containing a carotenoid-containing oily component are mixed, the carotenoid is inhibited from discoloring and both the dispersability and color of the emulsion particles may be stabilized. Furthermore, in a dispersion composition that further contains a fragrance agent, perfume-imparting properties of a fragrance agent may be favorably maintained.

Said at least ascorbic acid derivative may be present in an aqueous composition by an amount that may inhibit carotenoid from discoloring and may maintain dispersibility of emulsion particles when an aqueous composition is formed. Accordingly, an amount of said at least ascorbic acid derivative may be set at 0.1 to less than 10% by mass and preferably at 0.5 to 5% by mass or less relative to the mass of the whole aqueous composition, from the viewpoint of inhibiting carotenoids from decomposing.

The aqueous composition involving the invention may contain the foregoing oily component other than carotenoid. In one aspect of the invention, an aqueous composition contains an oily component other than carotenoid. When an oily component other than carotenoid is contained, the oily component is 20% by mass or less relative to the mass of the whole dispersion composition. The content is preferably in the range of 0.001 to 20% by mass, more preferably in the range of 0.001 to 10% by mass and particularly preferably in the range of 0.001 to 5% by mass. When the oily component is contained by an amount of 20% by mass or less in the dispersion composition, an oil phase and an aqueous phase may be readily suppressed from separating.

The aqueous composition involving the invention may contain polyols such as glycerin, PG (propylene glycol), BG (1,3-butylene glycol), pentylene glycol as an aqueous base material.

Furthermore, the aqueous composition involving the invention may contain various water-soluble polymer compounds and aqueous dispersible fine particles as a stabilizer of particles.

As the water-soluble polymer compound, all of synthetic polymers, natural polymers and semi-synthetic polymers may be used. Among these, sugars, proteins and composites thereof are preferred from the viewpoint of excellently stabilizing the carotenoid-containing oily component and so on.

The sugars include, but are not limited to, monosaccharides, disaccharides, oligosaccharides, polysaccharides, dextrin, starch derivatives, gums, mucopolysaccharides, celluloses and the like.

Among these, examples of typical sugars include, but are not limited to, agarose, arabinose, amylose, amylopectin, acacia gum, gum Arabic, arabinogalactan, alkyl glycoside, alginic acid, sodium alginate, alginic acid propylene glycol ester, aldose, inulin, oligosaccharide, gum gatti, curdlan, carrageenan, galactomannan, galactose, xanthan gum, xylose, xylogulcan, chitin, chitosan, guar gum, cluster dextrin, β-glucan, glucronic acid, glycogen, glycosaminoglycan, glyceric aldehyde, glucosamine, glucose, glucomannan, ketose, chondroitin sulfate, cyalume seed gum, jellan gum, cyclodextrin, sucrose, hydroxyethyl cellulose, hydroxypropyl cellulose, carboxymethyl cellulose, methyl cellulose, cellobiose, sorbitol, deoxyribose, dextrin, invert sugar, starch, soybean polysaccharide, sugar alcohol, sugar protein, tragacanth gum, trehalose, hyaluronic acid, fucose, fructose, pullulan, pectin, heparin, hemicellulose, maltose, mannitol, mannan, lactose, ribose, and the like.

Among the sugars, polysaccharides are preferred from the viewpoint of dispersion stability caused by an increase in the viscosity, and gum Arabic, pullulan or the like is more preferred from the viewpoint of stability of the carotenoids.

Furthermore, any kind of proteins may be used as long as it is a polymer or an oligomer in which amino acid is polymerized with a peptide bond. However, the proteins naturally derived and soluble in water are preferred.

There are a simple protein made of amino acid and a composite protein that contains a constituent other than amino acid. Both thereof may be used. Examples of simple protein include gelatin, collagen, casein, fibroin, sericin, keratin, protamine and so on. Examples of composite proteins include sugar protein that is a protein bonded to a carbohydrate, lipoprotein that is a protein bonded to a lipid, metal protein that is a protein bonded to a metal ion, nuclear protein that is a protein bonded to ribonucleic acid, phosphoprotein that is a protein bonded to a phosphate group, and the like.

On the other hand, generally, there are many proteins called from protein raw material. Examples thereof include animal muscle protein, milk protein, egg protein, rice protein, wheat protein (wheat gluten), soybean protein, yeast protein, bacterial protein and so on.

The proteins may be mixed and used.

Among the proteins, gelatin or soluble collagen is particularly preferred from the viewpoint of dispersion stability.

The stabilizer may be added at an arbitrary ratio related to the whole oily component, and it is preferably 0.1 times or more and 100 times or less, more preferably in the range of 0.5 times or more and 50 times or less, in order to stabilize the emulsion.

In the aqueous composition involving the invention, other than components particularly mentioned of the aqueous composition, among items described relating to the aqueous composition, a compound cited as a component capable of adding to the aqueous composition may be similarly contained. In this case, a ratio of amounts of the respective components may be used, as a total amount of each in the aqueous dispersion and aqueous composition, at amounts usually used to an entirety of the dispersion composition of the invention.

The dispersion composition of the invention is obtained by mixing the aqueous dispersion and the aqueous composition.

An average particle diameter of emulsion particles contained in the dispersion composition is 200 nm or less from the viewpoint of maintaining the transparency. Although an average particle diameter of emulsion particles in the dispersion composition may be larger than the particle diameter of the emulsion particles in the aqueous dispersion, it may be preferably set at 150 nm or less and more preferably at 100 nm or less from the viewpoint of maintaining the transparency and the dispersion stability. A measurement method of the emulsion particles in the dispersion composition may be conducted in a manner similar to the measurement of the emulsion particles in the aqueous dispersion.

The aqueous dispersion and the aqueous composition may be mixed without particular restriction as long as a small amount of the aqueous dispersion is added to a large amount of the aqueous composition so that the aqueous dispersion may be a part of the aqueous composition.

An addition amount of the aqueous dispersion to the aqueous composition may be appropriately varied depending on applications of the aqueous composition. The addition amount of the aqueous dispersion related to the whole dispersion composition is generally in the range of 0.01 to 10% by mass, preferably in the range of 0.05 to 5% by mass from the viewpoint of coloring density of the aqueous dispersion, and more preferably in the range of 0.1 to 1% by mass.

The dispersion composition of the invention in a second aspect of the invention is obtained by mixing a pH adjusting agent in addition to the aqueous dispersion and the aqueous composition. Herein, the dispersion composition according to the aspect has a pH value in the range of 5 to 7.5 and preferably 6.5 ~ 7.5. When the pH is adjusted in the range, the storage stability, in particular, the storage stability at room temperature is rendered excellent.

Herein, the room temperature in the invention means generally 10 to 40°C, preferably in the range of 15 to 30°C and in particular 25°C.

When the pH of the dispersion composition is adjusted, a pH adjusting agent may be appropriately blended to adjust. The pH adjusting agent may be any one of those that may be used in the usage, and inorganic acids, inorganic salts or organic acids, organic bases are cited. As the inorganic acid, hydrochloric acid, phosphoric acid, or carbonic acid may be preferably cited. As the inorganic salts, potassium hydroxide, sodium hydroxide, calcium hydroxide, magnesium hydroxide, disodium phosphate, monosodium phosphate, sodium carbonate, sodium hydrogen carbonate, or the like may be preferably cited. As the organic acids, without particular restriction, citric acid, trisodium citrate, gluconic acid, L-tartaric acid, malic acid, lactic acid, adipic acid, succinic acid, acetic acid, HEPES (2-[4-(2-Hydroxyethyl)-1-piperadinyl]ethanesulfonic acid) or derivatives thereof may be preferably cited but is not limited to. As the organic bases, glycine, lysine, guanidine, arginine, or trishydroxymethylaminomethane is cited. These may be used singularly or in a combination of at least two kinds thereof.

In the aspect where the pH of a dispersion composition is adjusted, a content of the pH adjusting agent in the dispersion composition may be an amount necessary for making the pH of the dispersion composition in the aforementioned range, and may be appropriately adjusted depending on the components in the dispersion composition and the kind of the pH adjusting agent used. However, the amount is generally in the range of 0.1 to 1.5% by mass and more preferably in the range of 0.5 to 1.0% by mass relative to the whole dispersion composition.

The dispersion composition of the invention may further contain a fragrance agent. The dispersion composition of the invention contains ascorbic acid or a derivative thereof in the aqueous composition; accordingly, perfuming properties of a fragrance agent may be stabilized and an odor from the carotenoid may be excellently masked.

As fragrance agents usable in the invention, any natural fragrance agents including animal-derived fragrances, plant-derived fragrances and mineral-derived fragrances and synthetic fragrance agents may be used. Examples thereof include, for example, rose-extracted essence, camomile-extracted essence, green tea perfume, lavender oil, geranium oil, jasmine oil, bergamot oil, musk oil, ylang-ylang oil, limonene, linalool, β-phenylethyl alcohol, 2,6-nonadienal, citral, cyclopentadecane, eugenol, rose oxide, indole, phenylacetaldehyde dimethyl acetal, auranthiol and the like. Among the fragrance agents, rose-extracted essence, camomile-extracted essence or green tea perfume are particularly preferred from the viewpoint of masking properties to odor of the carotenoid and perfuming properties.

The fragrance agents may be blended in the dispersion composition of the invention, and it is preferably blended in the aqueous composition from the viewpoint of dispersion stability. In this case, in the aqueous composition, the fragrance agent is blended, relative to addition mass of the aqueous composition to the aqueous dispersion, in the range of 1 to 50% by mass and preferably in the range of 5 to 20% by mass from the viewpoint of strength of the fragrance agent.

A dispersion composition of one aspect of the invention may be obtained as mentioned above according to a producing method that include mixing a astaxanthin and a phospholipid or a derivative thereof with an aqueous phase to obtain an aqueous dispersion comprising emulsion particles; and mixing the aqueous dispersion and an aqueous composition containing ascorbic acid derivative and an oily component that is 20% by mass or less relative to a mass of the whole dispersion composition.

Like this, when two-stages of mixing including the mixing for obtaining an aqueous dispersion and mixing of the resulted aqueous dispersion and the aqueous composition are undergone, a dispersion composition excellent in the storage stability, in which emulsion particles having an average particle diameter of 200 nm or less are dispersed, is readily obtained.

When a pH adjusting agent is blended to adjust the pH, the pH adjusting agent may preferably be blended in an aqueous dispersion from the viewpoint of the ease of adjusting the pH. However, as long as the pH of the aqueous dispersion is finally in the range, the pH adjusting agent may be added to any one of the dispersion composition, the aqueous composition and the aqueous dispersion, and may be added a plurality of times without particular restriction.

A cosmetic preparation for skin care of the invention contains the dispersion composition of the invention.

As mentioned above, the dispersion composition of the invention includes magnesium or sodium ascorbyl phosphate and is excellent in the storage stability; accordingly, a cosmetic preparation for skin care, which includes the dispersion composition, may similarly exhibit excellent storage stability.

In particular, a cosmetic preparation for skin care, which includes a dispersion composition having the pH value in the range of 5 to 7.5, may exhibit excellent storage stability at room temperature in addition to the characteristics.

Other components usable together with the dispersion composition are not particularly restricted. Components usually usable as a cosmetic preparation may be used as they are without particular restriction on the kind and blending amount.

EXAMPLES

In what follows, the present invention will be described with reference to examples.

However, the invention is not restricted thereto. In the following description, "parts" and "%" are based on mass unless clearly stated.

[Example 1]

(1) Preparation of Aqueous Dispersion (i)-a and (i)-b

The respective components described in Table 1 shown below were dissolved for 1 hr under heating at 70°C, and thereby an aqueous composition was obtained.

Furthermore, the respective components described in Table 2 shown below were dissolved for 1 hr under heating at 70°C, and thereby an oil phase composition was obtained.

[Table 1]

| Sucrose Stearate (HLB=16) | 13 g |
|---|---|
| Decaglyceryl Monooleate (HLB=12) | 25 g |
| Glycerin | 500 g |
| Pure Water | 322 g |
| Sucrose Stearate: RYOTO SUGAR ESTER S-1670 (HLB = 16)(trade name, manufactured by Mitsubishi-Kagaku Foods Corporation) Decaglyceryl Monooleate: NIKKOL DECAGLYN 1-O (HLB = 12) (trade name, manufactured by Nikko Chemicals Co., Ltd.) | |

[Table 2]

| Extract of Hematococcus Alga (containing 20% by mass of astaxanthins) | 40 g |
|---|---|
| Mix Tocopherol | 10 g |
| Lecithin (derived from soybean) | 90 g |
| Extract of Hematococcus Alga: ASTOTS-S (trade name, manufactured by Takedashiki Co., Ltd.), MIX TOCOPHEROL: RIKEN E OIL 800 (trade name, manufactured by Riken Vitamin Co., Ltd.), Lecithin (derived from soybean): LECION P (trade name, manufactured by Riken Vitamin Co., Ltd.) | |

With an aqueous phase kept at 70°C, a homogenizer (Vacuum Emulsification Device PVQ-1D (trade name, manufactured by Mizuho Kogyo Co., Ltd.)) was used to stir the aqueous phase (10000 rpm), and therein the oil phase composition was added to obtain an emulsion. The resulted emulsion was emulsified at 40°C under high-pressure of 200 MPa with ULTIMIZER HJP-25005 (trade name, manufactured by Sugino Machine Limited.).

Thereafter, a micro-filter having an average pore diameter of 1 μm was used to filter, thereby an astaxanthins-containing aqueous dispersion (i)-a was prepared.

An astaxanthins-containing aqueous dispersion (i)-b was similarly prepared except that 90 g of pure water was added in place of 90 g of lecithin in the oil phase composition.

(2) Preparation of Aqueous Compositions (ii)-a ~ (ii)-c

Components shown in Table 3 below were mixed and dissolved at room temperature and thereby aqueous compositions (ii)-a ~ (ii)-c were obtained.

[Table 3]

| Formulation | (ii)-a | (ii)-b | (ii)-c |
|---|---|---|---|
| Glycerin | 30 | 30 | 30 |
| BG | 20 | 20 | 20 |
| Xanthan Gum | 5 | 5 | 5 |
| Magnesium Ascorbyl Phosphate | 10 | 20 | - |
| Sodium Ascorbyl Phosphate | - | - | 10 |
| Ascorbyl-2-glucoside | - | - | - |
| Sodium Ascorbate | - | - | - |
| Ascorbic Acid | - | - | - |
| Methyl Paraben | 1.0 | 1.0 | 1.0 |
| Pure Water | 934 | 924 | 934 |
| Total (g) | 1000 | 1000 | 1000 |

(3) Preparation of Dispersion Composition

To 999.5 g of the aqueous composition (ii)-a, 0.5 g of an aqueous dispersion of a hematococcus alga extract (i)-a was added, followed by uniformly mixing, and thereby, a red transparent dispersion (A) was obtained as sample 1.
To 999 g of the aqueous composition (ii)-a, 1 g of an aqueous dispersion of a hematococcus alga extract (i)-a was added, followed by uniformly mixing, and thereby, a red transparent dispersion (B) was obtained as sample 2.
To 990 g of the aqueous composition (ii)-a, 10 g of an aqueous dispersion of a hematococcus alga extract (i)-a was added, followed by uniformly mixing, and thereby, a red transparent dispersion (C) was obtained as sample 3.
To 980 g of the aqueous composition (ii)-a, 20 g of an aqueous dispersion of a hematococcus alga extract (i)-a was added, followed by uniformly mixing, and thereby, a red transparent dispersion (D) was obtained as sample 4.
To 999 g of the aqueous composition (ii)-b, 1 g of an aqueous dispersion of a hematococcus alga extract (i)-a was added, followed by uniformly mixing, and thereby, a red transparent dispersion (E) was obtained as sample 5.
To 980 g of the aqueous composition (ii)-b, 20 g of an aqueous dispersion of a hematococcus alga extract (i)-a was added, followed by uniformly mixing, and thereby, a red transparent dispersion (F) was obtained as sample 6.
To 999 g of the aqueous composition (ii)-c, 1 g of an aqueous dispersion of a hematococcus alga extract (i)-a was added, followed by uniformly mixing, and thereby, a red transparent dispersion (G) was obtained as sample 7.
To 999 g of the aqueous composition (ii) d, 1 g of an aqueous dispersion of a hematococcus alga extrat (i)(a was added, followed by uniformly mixing, and thereby, a red transparent dispersion (H) was obtained as sample 8.
To 999 g of the aqueous comosition (ii)-e, 1 g of an aqueous dispersion of a hematococcus alga extract (i)-a was added, followed by uniformly mixing, and thereby, a red transparent dispersion (I) was obtained as sample 9.
To 999 g of the aqueous composition (ii)-f, 1 g of an aqueous dispersion of a hematococcus alga extract (i)-a was added, followed by uniformly mixing, and thereby, a red transparent dispersion (J) was obtained as sample 10.
To 999 g of the aqueous composition (ii)-g, 1 g of an aqueous dispersion of a hematococcus alga extract (i)-a was added, followed by uniformly mixing, and thereby, a red transparent dispersion (K) was obtained as sample 11.

(4) Measurement of Physicality Value

Each of the resulted dispersions (A) ~ (G) was charged in a light-shielding vessel, followed by closing with a cap, further followed by setting in a thermostat set at 50°C and leaving there for 30 days. Thereafter, a discoloring level (absorbance variation at λmax: 478 nm), a particle diameter, and a visual variation of state (turbidity, precipitation) of the dispersion were confirmed as shown below. Results thereof are shown in Table 4.

The absorbance was obtained by measuring an absorption spectrum at 478 nm with a UV-VIBLE SPECTROPHOTOMETER UV-2550 (trade name, manufactured by Shimadzu Corporation). A survival rate of the absorbance was obtained from the absorbance after 30 days at 50°C relative to the absorbance immediately after mixing an aqueous composition and aqueous dispersion. The survival rate of the absorbance was taken as a discoloring level of the dispersion and evaluated based on the following criteria.

A: The absorbance survival rate is 85% or more (excellent),
B: The absorbance survival rate is 70% or more (acceptable), and
C: The absorbance survival rate is less than 70% (inadequate).

As the particle diameter, a particle diameter of the emulsion in the dispersion was measured with a dynamic light scattering particle diameter distribution analyzer LB-550 (trade name, manufactured by Horiba Ltd.). A particle size variation between immediately after mixing an aqueous composition and an aqueous dispersion and after 30 days at 50°C was obtained and evaluated based on the criteria shown below.

A: 20 nm or less (excellent),
B: 70 nm or less (acceptable), and
C: exceeding 70 nm (inadequate)

The variation of state was visually observed in the aqueous composition after 30 days at 50°C and evaluated based on the evaluation criteria shown below.

A: A variation of state from immediately after mixing was not visually observed. (excellent)
B: A little turbidity was observed in the liquid. (acceptable)
C: The turbidity is enormous. The liquid is separated. Precipitation was caused. (inadequate)

[Table 4]

| Sample | Variation of Absorbance | | Particle Diameter | | | Visual Judgment of State | |
|---|---|---|---|---|---|---|---|
| | Absorbance Survival Rate at 478 nm | Judgment | Particle Diameter of Fresh Product | Particle Diameter Variation Δ, | Judgment | | |
| 1 | 94% | A | 48nm | +1nm | A | A | Example |
| 2 | 91% | A | 48nm | +1nm | A | A | Example |
| 3 | 90% | A | 52nm | +2nm | A | A | Example |
| 4 | 88% | A | 55nm | +3nm | A | A | Example |
| 5 | 97% | A | 49nm | +2nm | A | A | Example |
| 6 | 95% | A | 58nm | +2nm | A | A | Example |
| 7 | 87% | A | 50nm | +9nm | A | A | Example |
| 11 | 17% | C | 49nm | +2nm | A | A | Comparative Example |
| 12 | 82% | B | 82nm | +172nm | C | C | Comparative Example |

From the results, the dispersions involving examples of the invention were small in the average particle diameter immediately after emulsification and hardly showed variation in the particle diameter even after the storage. In visual observation of the emulsion after forced storage as well, the discoloration and variation of state were small and turbidity and precipitation were not observed.

As described above, according to the invention, aqueous compositions were provided that exhibited reduced variation over time in terms of the particle diameter, color and state, and excellent in the stability.

[Example 2]

(1) Preparation of Aqueous Dispersions (iii)-a and (iii)-b

Aqueous dispersions (iii)-a and (iii)-b were obtained in the same manner as the preparation of the aqueous dispersions (i)-a and (i)-b prepared in Example 1.

(2) Preparation of Aqueous Compositions (iv)-a ~ (iv)-c

Components shown in Table 5 shown below were mixed and dissolved at room temperature and thereby aqueous compositions (iv)-a ~ (iv)-c were obtained.

[Table 5]

| Prescription | (iv)-a | (iv)-b | (iv)-c |
|---|---|---|---|
| Glycerin | 30 | 30 | 30 |
| BG | 20 | 20 | 20 |
| Xanthan Gum | 5 | 5 | 5 |
| Magnesium Ascorbyl Phosphate | 10 | 20 | - |
| Sodium Ascorbyl Phosphate | - | - | 10 |
| Ascorbyl-2-glucoside | - | - | - |
| Sodium Ascorbate | - | - | - |
| Ascorbic Acid | - | - | - |
| Methyl Paraben | 1 | 1 | 1 |
| Pure Water | 834 | 824 | 834 |
| Total (g) | 900 | 900 | 900 |

In 900 g of the aqueous composition (iv)-a, a 1% aqueous solution of citric acid or a 0.1N aqueous solution of sodium hydroxide was added to adjust the pH to 4.5, followed by adjusting with pure water so that a total amount may be 990 g. Thereto, 10 g of an aqueous dispersion (iii)-a of hematococcus alga extract was added, followed by mixing uniformly, thereby a red transparent dispersion (A-1) was obtained. The pH of the dispersion (A-1) was substantially same as that before adding the aqueous dispersion (iii)-a (pH 4.5).

Furthermore, red transparent dispersions (A-2) to (A-9) were obtained in the same manner as the preparation of the dispersion (A-1) except that the pH was adjusted so as to be a value shown in Table 6.

Furthermore, a red transparent dispersion (H-1) was obtained in the same manner as the preparation of the dispersion (A-1) except that an aqueous composition (iv)-g was used in place of the aqueous composition (iv)-a and the pH was adjusted so as to be 7.0.

A red transparent dispersion (H-2) was obtained in the same manner as the preparation of the dispersion (A-1) except that an aqueous dispersion (iii)-b was used in place of the aqueous dispersion (iii)-a and the pH was adjusted so as to be 7.0.

(4) Measurement of Physicality Value

The resulted dispersions (A-1) to (A-9) and (H-1) to (H-2) were subjected to a time-lapse test as shown below.

(4-1) Time-lapse Test at 50°C

A sample was filled in a light-shielding vessel, followed by closing a cap, further followed by storing for 28 days in a thermostat set at 50°C.

(4-2) Air Bubble Time-lapse Test at 25°C

A sample was filled in a light shielding vessel and, in a thermostat set at 25°C, air was flowed at a flow rate of 1 cc/min with a 3 mm φ glass tube. The test was continued for 28 days with measuring the mass of the samples every one week and a volatile component compensating with pure water.

(4-3) Nitrogen Bubbling Time-lapse Test at 25°C

A sample was filled in a light shielding vessel and, in a thermostat set at 25°C, nitrogen was flowed at a flow rate of 1 cc/min with a 3 mm φ glass tube. The test was continued for 28 days with a mass thereof measuring every one week and a volatile component compensating with pure water.

At the measurement of the physicality values, the discoloring level was judged based on the variation of absorbance at 478 nm, and variation of state was confirmed from visual variation (turbidity, precipitation) and from particle diameter.

Results are shown in Table 6.

The absorbance was obtained by measuring an absorption spectrum at 478 nm with a UV-VISIBLE SPECTROPHOTOMETER UV-2550 (trade name, manufactured by Shimadzu Corporation). A survival rate of the absorbance was obtained from the absorbance after the time-lapse test relative to the absorbance immediately after mixing an aqueous composition and aqueous dispersion. The survival rate of the absorbance was taken as a discoloring level of the dispersion and evaluated based on the following criteria.

A: The absorbance survival rate was 85% or more (a level where variation was not observed on a palm and there was no problem)
B: The absorbance survival rate was 70% or more (a level where variation was observed on a palm but there was no problem of commodity value)
C: The absorbance survival rate was less than 70% (inadequate)

A particle diameter of the emulsion in the dispersion was measured with a dynamic light scattering particle diameter distribution analyzer LB-550 (trade name, manufactured by Horiba Ltd.) at 20°C. A particle size variation between immediately after mixing an aqueous composition and an aqueous dispersion and after the time-lapse test was obtained and evaluated based on the criteria shown below.

A: 20 nm or less (excellent),
B: 70 nm or less (acceptable), and
C: exceeding 70 nm (inadequate)

The visual variation of state was visually observed of the aqueous composition after time-lapse test and evaluated based on the evaluation criteria shown below.

A: A visual variation of state from immediately after mixing was not observed. (excellent)
B: A little turbidity was observed in the liquid. (acceptable)
C: The turbidity is enormous. The liquid is separated. Precipitation is caused. (inadequate)

[Table 6]

| Example 2 | | | | | | | | |
|---|---|---|---|---|---|---|---|---|
| Sample | Particle Diameter (nm) | pH | Absorbance | | | State | | Note |
| | | | 50°C | 25°C (air) | 25°C (nitrogen) | Visual | Particle Diameter | |
| A-1 | 78 | 4.5 | B | C | B | A | A | Comparative Example |
| A-2 | 78 | 5.0 | A | B | A | A | A | Example |
| A-3 | 75 | 5.5 | A | B | A | A | A | Example |
| A-4 | 73 | 6.0 | A | B | A | A | A | Example |
| A-5 | 68 | 6.5 | A | A | A | A | A | Example |
| A-6 | 68 | 7.0 | A | A | A | A | A | Example |

(continued)

| Example 2 | | | | | | | | | |
|---|---|---|---|---|---|---|---|---|---|
| Sample | Particle Diameter (nm) | pH | Absorbance | | | State | | | Note |
| | | | 50°C | 25°C (air) | 25°C (nitrogen) | Visual | Particle Diameter | | |
| A-7 | 69 | 7.5 | A | A | A | A | A | | Example |
| A-8 | 72 | 8.0 | A | C | B | A | A | | Comparative Example |
| A-9 | 75 | 8.5 | B | C | B | A | A | | Comparative Example |
| H-1 | 65 | 7.0 | C | C | B | A | A | | Comparative Example |
| H-2 | 350 | 7.0 | A | B | A | C | C | | Comparative Example |

From the foregoing results, dispersions (A-2 to A-7) having pH 5 to 7.5 of examples involving one aspect of the invention were small in the average particle diameters immediately after emulsification and hardly showed particle diameter variation even after the time-lapse storage. In visual observation of emulsion after forced time-lapse storage as well, the discoloring variation and state variation were small, and turbidity and precipitation were not observed. In particular, in the time-lapse storage at 25°C, an extent of the discoloration was small and the stability was excellent compared with comparative examples, and it was remarkable in the pH 6.5 to 7.5.

[Examples 3 to 7]

Each of dispersions B-1 to B-9 (Example 3), C-1 to C-9 (Example 4), D-1 to D-9 (Example 5), E-1 to E-9 (Example 6) and F-1 to F-9 (Example 7) was prepared in the same manner as the preparation the samples in Example 1 except that each of (iv)-b, (iv)-c, (iv)-d, (iv)-e, and (iv)-f was used in place of the aqueous composition (iv)-a. Each of the dispersions was subjected to evaluations same as Example 2. Results of Examples 3 to 7 are shown in Tables 7 to 11, respectively.

As the results thereof, in all of Examples 3 to 7, similarly to Example 1, in a dispersion of which pH is 5 to 7.5, an average particle diameter immediate after emulsification was small and hardly showed variation even after the time-lapse storage. In the visual observation of the emulsion after forced time-lapse storage as well, the discoloration variation and state variation were small and the turbidity and precipitation were not observed. In particular in the time-lapse storage at 25°C, a degree of discoloration was small and the stability was excellent more than Comparative Examples in particular in the pH 6.5 to 7.5.

[Table 7]

| Example 3 | | | | | | | | | |
|---|---|---|---|---|---|---|---|---|---|
| Sample | Particle Diameter (mn) | pH | Absorbance | | | State | | | Note |
| | | | 50°C | 25°C (air) | 25°C (nitrogen) | Visual | Particle Diameter | | |
| B-1 | 78 | 4.5 | B | C | B | A | A | | Comparative Example |
| B-2 | 77 | 5.0 | A | B | A | A | A | | Example |
| B-3 | 74 | 5.5 | A | A | A | A | A | | Example |
| B-4 | 73 | 6.0 | A | A | A | A | A | | Example |
| B-5 | 69 | 6.5 | A | A | A | A | A | | Example |
| B-6 | 68 | 7.0 | A | A | A | A | A | | Example |
| B-7 | 68 | 7.5 | A | A | A | A | A | | Example |

(continued)

| Example 3 | | | | | | | | | |
|---|---|---|---|---|---|---|---|---|---|
| Sample | Particle Diameter (mn) | pH | Absorbance | | | State | | | Note |
| | | | 50°C | 25°C (air) | 25°C (nitrogen) | Visual | Particle Diameter | | |
| B-8 | 73 | 8.0 | A | C | A | A | A | | Comparative Example |
| B-9 | 79 | 8.5 | B | C | B | A | A | | Comparative Example |
| H-1 | 65 | 7.0 | C | C | B | A | A | | Comparative Example |
| H-2 | 350 | 7.0 | A | B | A | C | C | | Comparative Example |

[Table 8]

| Example 4 | | | | | | | | | |
|---|---|---|---|---|---|---|---|---|---|
| Sample | Particle Diameter (nm) | pH | Absorbance | | | State | | | Note |
| | | | 50°C | 25°C (air) | 25°C (nitrogen) | Visual | Particle Diameter | | |
| C-1 | 76 | 4.5 | B | C | B | A | A | | Comparative Example |
| C-2 | 79 | 5.0 | A | B | A | A | A | | Example |
| C-3 | 76 | 5.5 | A | B | A | A | A | | Example |
| C-4 | 76 | 6.0 | A | B | A | A | A | | Example |
| C-5 | 70 | 6.5 | A | A | A | A | A | | Example |
| C-6 | 70 | 7.0 | A | A | A | A | A | | Example |
| C-7 | 69 | 7.5 | A | A | A | A | A | | Example |
| C-8 | 76 | 8.0 | A | C | B | A | A | | Comparative Example |
| C-9 | 78 | 8.5 | B | C | B | A | A | | Comparative Example |
| H-1 | 65 | 7.0 | C | C | B | A | A | | Comparative Example |
| H-2 | 350 | 7.0 | A | B | A | C | C | | Comparative Example |

[Table 9]

| Example 5 | | | | | | | | | |
|---|---|---|---|---|---|---|---|---|---|
| Sample | Particle Diameter (nm) | pH | Absorbance | | | State | | | Note |
| | | | 50°C | 25°C (air) | 25°C (nitrogen) | Visual | Particle Diameter | | |
| D-1 | 79 | 4.5 | B | C | B | B | A | | Comparative Example |

(continued)

| Example 5 | | | | | | | | | |
|---|---|---|---|---|---|---|---|---|---|
| Sample | Particle Diameter (nm) | pH | Absorbance | | | State | | | Note |
| | | | 50°C | 25°C (air) | 25°C (nitrogen) | Visual | Particle Diameter | | |
| D-2 | 76 | 5.0 | A | B | B | A | A | | Example |
| D-3 | 73 | 5.5 | A | B | A | A | A | | Example |
| D-4 | 74 | 6.0 | A | B | A | A | A | | Example |
| D-5 | 65 | 6.5 | A | A | A | A | A | | Example |
| D-6 | 69 | 7.0 | A | A | A | A | A | | Example |
| D-7 | 66 | 7.5 | A | A | A | A | A | | Example |
| D-8 | 73 | 8.0 | A | C | B | A | A | | Comparative Example |
| D-9 | 76 | 8.5 | B | C | B | A | A | | Comparative Example |
| H-1 | 65 | 7.0 | C | C | B | A | A | | Comparative Example |
| H-2 | 350 | 7.0 | A | B | A | C | C | | Comparative Example |

[Table 10]

| Example 6 | | | | | | | | | |
|---|---|---|---|---|---|---|---|---|---|
| Sample | Particle Diameter (nm) | pH | Absorbance | | | State | | | Note |
| | | | 50°C | 25°C (air) | 25°C (nitrogen) | Visual | Particle Diameter | | |
| E-1 | 79 | 4.5 | B | C | B | B | A | | Comparative Example |
| E-2 | 80 | 5.0 | A | B | B | B | A | | Example |
| E-3 | 80 | 5.5 | A | B | B | A | A | | Example |
| E-4 | 76 | 6.0 | A | B | B | A | A | | Example |
| E-5 | 73 | 6.5 | A | B | A | A | A | | Example |
| E-6 | 73 | 7.0 | A | A | A | A | A | | Example |
| E-7 | 76 | 7.5 | A | B | A | A | A | | Example |
| E-8 | 77 | 8.0 | A | C | B | A | A | | Comparative Example |
| E-9 | 80 | 8.5 | B | C | B | A | A | | Comparative Example |
| H-1 | 65 | 7.0 | C | C | B | A | A | | Comparative Example |
| H-2 | 350 | 7.0 | A | B | A | C | C | | Comparative Example |

[Table 11]

| Example 7 | | | | | | | | |
|---|---|---|---|---|---|---|---|---|
| Sample | Particle Diameter (mn) | pH | Absorbance | | | State | | Note |
| | | | 50°C | 25°C (air) | 25°C (nitrogen) | Visual | Particle Diameter | |
| F-1 | 83 | 4.5 | B | C | B | B | A | Comparative Example |
| F-2 | 82 | 5.0 | A | B | B | B | A | Example |
| F-3 | 81 | 5.5 | A | B | B | A | A | Example |
| F-4 | 77 | 6.0 | A | B | B | A | A | Example |
| F-5 | 73 | 6.5 | A | B | A | A | A | Example |
| F-6 | 73 | 7.0 | A | A | A | A | A | Example |
| F-7 | 75 | 7.5 | A | B | A | A | A | Example |
| F-8 | 76 | 8.0 | A | C | B | A | A | Comparative Example |
| F-9 | 80 | 8.5 | B | C | B | A | A | Comparative Example |
| H-1 | 65 | 7.0 | C | C | B | A | A | Comparative Example |
| H-2 | 350 | 7.0 | A | B | A | C | C | Comparative Example |

As mentioned above, according to the invention, an aqueous composition is provided that exhibits reduced variation over time in terms of particle diameter, color and state, that is, that is excellent in storage stability and, in particular, storage stability at room temperature.

Further the aqueous composition may be used to provide a cosmetic preparation for skin care having excellent storage stability.

An entirety of disclosures of Japanese Patent Application Nos. 2007-679 and 2007-169635 is incorporated in the present specification by reference.

**Claims**

1.    A dispersion composition comprising

(a) emulsion particles having an average particle diameter of 200 nm or less and containing astaxanthin and a phospholipid;
(b) at least one ascorbic acid derivative selected from magnesium ascorbyl phosphate and sodium ascorbyl phosphate; and
(c) an oily component that is 20% by mass or less relative to the mass of the whole dispersion composition.

2.    The dispersion composition according to claim 1, wherein the phospholipid is lecithin.

3.    The dispersion composition according to claim 1 or claim 2, further comprising tocopherol.

4.    The dispersion composition according to, any one of claims 1 to 3 wherein the astaxanthin is derived from an extract of *Hematococcus* alga.

5.    The dispersion composition according to any one of claims 1 to 4, further comprising glycerin.

**6.** A cosmetic preparation for skin care comprising the dispersion composition according to any one of claims 1 to 5.

**7.** A method for producing a dispersion composition which is the dispersion composition according to any one of claims 1 to 5, the method comprising:

mixing a carotenoid-containing oily component containing astaxanthin and a phospholipid with an aqueous phase to obtain an aqueous dispersion containing emulsion particles; and
mixing the aqueous dispersion and an aqueous composition containing at least one ascorbic acid derivative selected from sodium ascorbyl phosphate and magnesium ascorbyl phosphate and an oily component that is 20% by mass or less relative to the mass of the whole dispersion composition to obtain a dispersion composition comprising emulsion particles having an average particle diameter of 200 nm or less.

**8.** A method for producing a dispersion composition according to claim 7, wherein the content of the phospholipid is from 0.001% mass to 20% by mass relative to the mass of the whole aqueous dispersion.

**9.** A cosmetic preparation for skin care according to claim 6 having a pH of from 5.0 to 7.5.

**10.** The cosmetic preparation for skin care according to claim 9, wherein the phospholipid is lecithin.

**11.** The cosmetic preparation for skin care according to claim 9 or claim 10, further comprising tocopherol.

**12.** The cosmetic preparation for skin care according to any one of claims 10 to 11, further comprising glycerin.

**13.** The cosmetic preparation for skin care according to claim 12, wherein the content of glycerin is from 10% by mass to 60% by mass relative to the mass of the whole cosmetic preparation for skin care.

**14.** The cosmetic preparation for skin care according to any one of claims 9 to 13, wherein the average particle diameter of the emulsion particles is 200 nm or less.

**15.** A method for producing a cosmetic composition for skin care according to claim 7, the method further comprising:

adjusting the pH of the dispersion composition to the range of 5 to 7.5.

**16.** The method for producing a cosmetic preparation for skin care according to claim 15, wherein the content of the phospholipid is from 0.001% mass to 20% by mass relative to the mass of the whole aqueous dispersion.

**Patentansprüche**

**1.** Dispersionszusammensetzung, die Folgendes umfasst:

(a) Emulsionsteilchen mit einem durchschnittlichen Teilchendurchmesser von 200 nm oder weniger und die Astaxanthin und ein Phospholipid enthalten;
(b) mindestens ein Ascorbinsäurederivat, ausgewählt aus Magnesiumascorbylphosphat und Natriumascorbylphosphat; und
(c) eine Ölkomponente, die 20 Masse-% oder weniger in Bezug auf die Masse der gesamten Dispersionszusammensetzung beträgt.

**2.** Dispersionszusammensetzung nach Anspruch 1, wobei es sich bei dem Phospholipid um Lecithin handelt.

**3.** Dispersionszusammensetzung nach Anspruch 1 oder 2, die weiterhin Tocopherol umfasst.

**4.** Dispersionszusammensetzung nach einem der Ansprüche 1 bis 3, wobei das Astaxanthin aus einem Extrakt der Alge *Hematococcus* stammt.

**5.** Dispersionszusammensetzung nach einem der Ansprüche 1 bis 4, die weiterhin Glycerin umfasst.

**6.** Kosmetikpräparat für die Hautpflege, das die Dispersionszusammensetzung nach einem der Ansprüche 1 bis 5 umfasst.

7. Verfahren zur Herstellung einer Dispersionszusammensetzung, bei der es sich um die Dispersionszusammensetzung nach einem der Ansprüche 1 bis 5 handelt, wobei das Verfahren Folgendes umfasst:

Mischen einer carotinhaltigen Ölkomponente, die Astaxanthin und ein Phospholipid enthält, mit einer wässrigen Phase, wodurch man zu einer wässrigen Dispersion, die Emulsionsteilchen enthält, gelangt; und
Mischen der wässrigen Dispersion und einer wässrigen Zusammensetzung, die mindestens ein Ascorbinsäurederivat, ausgewählt aus Natriumascorbylphosphat und Magensiumascorbylphosphat, enthält, und einer Ölkomponente, die 20 Masse-% oder weniger in Bezug auf die Masse der gesamten Dispersionszusammensetzung ausmacht, wodurch man zu einer Dispersionszusammensetzung, die Emulsionsteilchen mit einem durchschnittlichen Teilchendurchmesser von 200 nm oder weniger umfasst, gelangt.

8. Verfahren zur Herstellung einer Dispersionszusammensetzung nach Anspruch 7, wobei der Phospholipidgehalt von 0,001 Masse-% bis 20 Masse-% in Bezug auf die Masse der gesamten wässrigen Dispersion beträgt.

9. Kosmetikpräparat für die Hautpflege nach Anspruch 6, die einen pH-Wert von 5,0 bis 7,5 aufweist.

10. Kosmetikpräparat für die Hautpflege nach Anspruch 9, wobei es sich bei dem Phospholipid um Lecithin handelt.

11. Kosmetikpräparat für die Hautpflege nach Anspruch 9 oder Anspruch 10, das weiterhin Tocopherol umfasst.

12. Kosmetikpräparat für die Hautpflege nach einem der Ansprüche 10 bis 11, das weiterhin Glycerin umfasst.

13. Kosmetikpräparat für die Hautpflege nach Anspruch 12, wobei der Glyceringehalt von 10 Masse-% bis 60 Masse-% auf die Masse des gesamten Kosmetikprodukts für die Hautpflege beträgt.

14. Kosmetikpräparat für die Hautpflege nach einem der Ansprüche 9 bis 13, wobei der durchschnittliche Teilchendurchmesser der Emulsionsteilchen 200 nm oder weniger beträgt.

15. Verfahren zur Herstellung einer Kosmetikzusammensetzung für die Hautpflege nach Anspruch 7, wobei das Verfahren weiterhin Folgendes umfasst:

Einstellen des pH-Werts der Dispersionszusammensetzung in dem Bereich von 5 bis 7,5.

16. Verfahren zur Herstellung einer Kosmetikzusammensetzung für die Hautpflege nach Anspruch 15, wobei der Phospholipidgehalt von 0,001 Masse-% bis 20 Masse-% in Bezug auf die Masse der gesamten wässrigen Dispersion beträgt.

**Revendications**

1. Composition en dispersion, comprenant

(a) des particules d'émulsion ayant un diamètre de particules moyen de 200 nm ou moins et contenant de l'astaxanthine et un phospholipide ;
(b) au moins un dérivé d'acide ascorbique choisi parmi le phosphate d'ascorbyle de magnésium et le phosphate d'ascorbyle de sodium ; et
(c) un composant huileux qui constitue 20% en masse ou moins par rapport à la masse de l'ensemble de la composition en dispersion.

2. Composition de dispersion selon la revendication 1, dans laquelle le phospholipide est la lécithine.

3. Composition de dispersion selon la revendication 1 ou 2, comprenant en outre du tocophérol.

4. Composition de dispersion selon l'une quelconque des revendications 1 à 3, dans laquelle l'astaxanthine est dérivée d'un extrait d'algue *Haematococcus.*

**5.** Composition de dispersion selon l'une quelconque des revendications 1 à 4, comprenant en outre du glycérol.

**6.** Préparation cosmétique pour le soin de la peau, comprenant la composition en dispersion selon l'une quelconque des revendications 1 à 5.

**7.** Méthode de production d'une composition en dispersion qui est la composition en dispersion selon l'une quelconque des revendications 1 à 5, la méthode comprenant :

le mélange d'un composant huileux contenant des caroténoïdes contenant de l'astaxanthine et un phospholipide, avec une phase aqueuse afin d'obtenir une dispersion aqueuse contenant des particules d'émulsion ; et le mélange de la dispersion aqueuse et d'une composition aqueuse contenant au moins un dérivé d'acide ascorbique choisi parmi le phosphate d'ascorbyle de sodium et le phosphate d'ascorbyle de magnésium, et un composant huileux qui constitue 20% en masse ou moins par rapport à la masse de l'ensemble de la composition en dispersion, afin d'obtenir une composition en dispersion comprenant des particules d'émulsion ayant un diamètre de particules moyen de 200 nm ou moins.

**8.** Méthode de production d'une composition en dispersion selon la revendication 7, dans laquelle la teneur en phospholipide va de 0,001% en masse à 20% en masse par rapport à la masse de l'ensemble de la dispersion aqueuse.

**9.** Préparation cosmétique pour le soin de la peau selon la revendication 6, ayant un pH allant de 5,0 à 7,5.

**10.** Préparation cosmétique pour le soin de la peau selon la revendication 9, dans laquelle le phospholipide est la lécithine.

**11.** Préparation cosmétique pour le soin de la peau selon la revendication 9 ou la revendication 10, comprenant en outre du tocophérol.

**12.** Préparation cosmétique pour le soin de la peau selon l'une quelconque des revendications 10 à 11, comprenant en outre du glycérol.

**13.** Préparation cosmétique pour le soin de la peau selon la revendication 12, dans laquelle la teneur en glycérol va de 10% en masse à 60% en masse par rapport à la masse de l'ensemble de la préparation cosmétique pour le soin de la peau.

**14.** Préparation cosmétique pour le soin de la peau selon l'une quelconque des revendications 9 à 13, dans laquelle le diamètre de particules moyen des particules d'émulsion est de 200 nm ou moins.

**15.** Méthode de production d'une composition cosmétique pour le soin de la peau selon la revendication 7, la méthode comprenant en outre :

l'ajustement du pH de la composition en dispersion dans la plage allant de 5 à 7,5.

**16.** Méthode de production d'une préparation cosmétique pour le soin de la peau selon la revendication 15, dans laquelle la teneur en phospholipide va de 0,001% en masse à 20% en masse par rapport à la masse de l'ensemble de la dispersion aqueuse.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2049091 A
- JP 9143063 A
- JP 5155736 A
- JP 2005047860 A
- JP 9328419 A
- JP 2005506841 A
- JP 8103288 A
- JP 5068585 A
- JP 2007000679 A
- JP 2007169635 A

### Non-patent literature cited in the description

- **DAVIES, B. H.** Chemistry and Biochemistry of Plant Pigments. Academic Press, 1976, 38-165
- **YAMAGUCHI, K. ; MIKI, W. ; TORIU, N. ; KONDO, Y. ; MURAKAMI, M. ; KONOSU, S. ; SATAKE, M. ; FUJITA, T.** The composition of carotenoid colorants in the antrarctic krill Euphausia superba. *Bull. Jap. Sos. Sci. Fish.,* 1983, vol. 49, 1411-1415
- **RENSTROM, B. ; LIAAEN-JENSEN, S.** Fatty acids of some estrified carotenols. *Comp. Biochem. Physiol. B, Comp. Biochem.,* 1981, vol. 69, 625-627
- **ANDREWES, A.G. ; STARR, M. P.** 3R,3'R)-Astraxanthin from the yeast Phaffarhodozyma. *Phytochem.,* 1976, vol. 15, 1009-1011
- **ANDREWES, A.G. ; PHAFFIA, H. J. ; STARR, M. P.** Carotenids of Phaffia rhodozyma, a red pigmented fermenting yeast. *Phytochem.,* 1976, vol. 15, 1003-1007
- **KUHN, R. ; SOERENSEN, N. A.** The coloring matters of the lobster (Astracus gammarus L.). *Z. Angew. Chem.,* 1938, vol. 51, 465-466
- **CHEESMAN, D. F.** Ovorubin, a chromoprotein from the eggs of the gastropod mollusc Pomacea canaliculata. *Proc. Roy. Soc. B,* 1958, vol. 149, 571-587
- Oil Chemistry Handbook. Japan Oil Chemists' Association, 2001
- **KAJIMOTO ; SAN SHOBO.** *Theory and Fact of Antioxidant,* 1984
- **SARUWATARI ; NISHINO ; TABATA ; TAISEI-SHA.** Antioxidant Handbook. 1976